# EUROPEAN PATENT APPLICATION

(11) **EP 4 550 343 A1**
(43) Date of publication of application: **07.05.2025**
(21) Application number: 24209941.4
(22) Date of filing: 30.10.2024
(51) Int. Cl.: G16H 10/60, A61B 5/00, G16H 40/67

(54) **MEDICAL DEVICE DATA INTERCONNECTIVITY IN A HEALTHCARE NETWORK**

(30) Priority: 31.10.2023 US 202363594489 P; 12.09.2024 US 202463693762 P
(71) Applicant: Hill-Rom Services, Inc., Batesville, IN 47006-9167 (US)
(72) Inventor: WILLIAMS, Jason M, Batesville, 47006-9167 (US)
(74) Representative: Reddie & Grose LLP

(57) **Abstract**

A system is provided for automatically provisioning devices in a localized wireless network. The system includes an outlet. The outlet is configured to provision a plurality of electronic devices so that a first electronic device is enabled to communicate with a second electronic device.

## Description

### BACKGROUND

The present disclosure generally relates to localized wireless networks, and more particularly, to a system and method for automatically provisioning a medical device onto a localized wireless network.

A healthcare setting typically includes a plurality of medical devices. When a patient is admitted into the healthcare setting, patient treatment often requires several medical devices that are brought into the patient's room. When medical devices are in proximity to each other and centered around a patient, there is a need for the devices to share data with each other. There is also a need for a localized network to exchange data related to the patient and the medical devices. Generally, a caregiver would not want to manually configure this network, and therefore the network should form without user intervention. The current prior art systems and devices do not adequately address the above-mentioned needs.

Also, medical devices often contain information and features that would be useful to other medical devices that lack that information or capability. As a result, data entry or necessary hardware must be duplicated on each device requiring the information. For example, a patient support apparatus may be capable of measuring a patient's weight and such information may be useful at a vitals monitor or an intravenous (IV) pump (aka infusion pump) in the patient room. In another example, the patient support apparatus may have circuitry that transmits alert messages to a nurse call system and a nearby IV pump, which is not in communication with the nurse call system, may have a bag or other container of IV fluid that is near empty. Accordingly, there is an ongoing need for medical devices to share information with each another or to share their capabilities with other medical devices in order to be able to operate more effectively in caring for patients and/or providing notifications to nurse call systems.

### SUMMARY

The present disclosure includes one or more of the features recited in the appended claims and/or the following features which, alone or in any combination, may comprise patentable subject matter.

According to a first aspect of the disclosed embodiments, a system for automatically provisioning devices in a localized wireless network includes a plurality of power receptacles. Each power receptacle includes a plug detector in communication with an outlet processor of an outlet. An outlet communication module is located in the outlet and in communication with the processor. Each power receptacle is configured to provision an electronic device. The electronic device has a device processor and a device communication module in communication with the device processor. In response to a first power plug of a first electronic device being inserted into a first power receptacle of the plurality of power receptacles and detected by a first plug detector associated with the first power receptacle, the outlet processor instructs the outlet communication module to communicate with the device communication module of first electronic device to add the first electronic device to a localized wireless network. In response to a second power plug of a second electronic device being inserted into a second power receptacle of the plurality of power receptacles and detected by a second plug detector associated with the second power receptacle, the outlet processor instructs the outlet communication module to communicate with the device communication module of second electronic device to add the second electronic device to the localized wireless network. The device communication module of the first electronic device is enabled to communicate with the device communication module of the second electronic device through the localized wireless network.

In some embodiments of the first aspect, each power receptacle may include an indicator configured to represent a status of the power receptacle. The indicator may include a multi-colored light. The status may include a connection status of the outlet communication module. The outlet processor may be configured to update the indicator based on the status of the power receptacle.

Optionally, in the first aspect, the outlet communication module may be wireless. The outlet communication module may communicate with the first device communication module and the second device communication module wirelessly. The outlet processor may be configured to detect the insertion of a power plug into one of the plurality of power receptacles with the associated plug detector. The outlet processor may be configured to exchange data with the first electronic device and the second electronic device. The device processor of each of the plurality of electronic devices may be configured to exchange data with the outlet processor over the localized wireless network. The device processor of each of the first electronic device and the second electronic device may be configured to exchange data with the device processor of other electronic device of the plurality of electronic devices over the localized wireless network.

In may be desired, in the first aspect, that the outlet establishes communication with each of the first electronic device and the second electronic device by generating and sending provisioning data, in response to the respective power plug being inserted into one of the plurality of power receptacles and detected by the associated plug detector. Each of the first electronic device and the second electronic device may establish communication with the outlet by sending a signal to the outlet processor indicating an availability to connect, in response to the respective power plug being inserted into one of the plurality of power receptacles and detected by the associated plug detector. The outlet may generate and send a provisioning invite to the respective electronic device, in response to receiving the signal from the respective electronic device. The outlet and the respective electronic device may exchange public keys, in response to the respective electronic device receiving the provisioning invite. A cryptographic exchange may occur between the outlet and the respective electronic device, in response to the exchange of public keys. The outlet may generate and send provisioning data to the respective electronic device, in response to the cryptographic exchange.

In some embodiments of the first aspect, each of the first electronic device and the second electronic device may include a medical device. The outlet may include a power outlet in a healthcare facility. Each power receptacle may include an alternating current power receptacle.

According to a second aspect of the disclosed embodiments, a method for automatically provisioning devices in a localized wireless network includes. The method also includes creating a localized wireless network between a plurality of power receptacles. Each power receptacle is associated with an outlet communication module. Creating the localized wireless network includes generating communication between the plurality of power receptacles. The method also includes detecting the insertion of a first power plug of a first electronic device into a first power receptacle of the plurality of power receptacles. The method also includes generating communication between the outlet communication module and the first electronic device to add the first electronic device to the localized wireless network. The method also includes detecting the insertion of a second power plug of a second electronic device into a second power receptacle of the plurality of power receptacles. The method also includes generating communication between the outlet communication module and the second electronic device to add the second electronic device to the localized wireless network. The method also includes providing communication between the first electronic device and the second electronic device through the localized wireless network.

In some embodiments of the second aspect, the method may also include establishing communication between the outlet communication module and each of the first electronic device and the second electronic device by generating and sending provisioning data from the outlet communication module to the first electronic device and the second electronic device, in response to the respective power plug being inserted into one of the plurality of power receptacles. The method may also include establishing communication between the localized wireless network and each of the first electronic device and the second electronic device by sending a signal from the respective electronic device to the outlet communication module indicating an availability to connect, in response to the respective power plug being inserted into one of the plurality of power receptacles. The method may also include generating and sending a provisioning invite from the outlet communication module to the respective electronic device, in response to receiving the signal from the respective electronic device. The method may also include exchanging public keys between the outlet communication module and the respective electronic device, in response to the respective electronic device receiving the provisioning invite. The method may also include generating a cryptographic exchange between the outlet communication module and the respective electronic device, in response to the exchange of public keys. The method may also include generating and sending provisioning data from the outlet communication module to the respective electronic device, in response to the cryptographic exchange.

Optionally, in the second aspect, the method may also include updating an indicator associated with each power receptacle based on a status of the power receptacle. The method may also include exchanging data between the outlet communication module and each of the first electronic device and the second electronic device over the localized wireless network. The method may also include exchanging data between the first electronic device and the second electronic device over the localized wireless network.

In some embodiments of the second aspect, at least one of the first electronic device and the second electronic device may include a medical device. Each power receptacle may include a power receptacle in a healthcare facility. Each power receptacle may include an alternating current power receptacle.

According to a third aspect of the disclosed embodiments, a localized wireless network includes a first medical device. A first power receptacle is configured to provide power via a cord to the first medical device. The first power receptacle is associated with a wireless communicator to provide wireless communication between the receptacle and the first medical device. A second medical device is provided. A second power receptacle is configured to provide power via a cord to the second medical device. The second power receptacle is associated with a wireless communicator to provide wireless communication between the receptacle and the second medical device. The network is configured such that the first medical device communicates wirelessly with the second medical device.

According to a fourth aspect of the disclosed embodiments, a localized wireless network includes a first medical device having at least one sensor for acquiring data related to a patient. A first power receptacle is configured to provide power via a cord to the first medical device. The first power receptacle is associated with a wireless communicator to provide wireless communication between the first power receptacle and the first medical device. A second medical device is provided. A second power receptacle is configured to provide power via a cord to the second medical device. The second power receptacle is associated with a wireless communicator to provide wireless communication between the second power receptacle and the second medical device. The network is configured such that the first medical device communicates wirelessly with the second medical device. The first medical device is configured to publish the data related to the patient on the network. The second medical device is configured to receive the data from the network.

In some embodiments of the fourth aspect, the second medical device may be connected to an electronic medical record. The second medical device may be wirelessly connected to the electronic medical record. The second medical device may include a scanner configured to scan a patient code to connect to the electronic medical record. The electronic medical record may be specifically linked to the patient via the patient code. The patient code may be provided on a patient wristband worn by the patient. The second medical device may be configured to publish the data to the electronic medical record.

Optionally, in the fourth aspect, the first medical device may include a patient support apparatus. The second medical device may include at least one of a physiological monitor and an infusion pump. The data may include services provided by the first medical device. The services may include at least one of an electronic medical record channel, a service-oriented device connectivity communication channel, nurse call capabilities, and priority nurse call capabilities. The data may include at least one of patient weight, patient identification, patient blood pressure, patient heart rate, patient respiratory rate, lighting status, caregiver presence indication, and caregiver identification.

It may be desired, in the fourth aspect, that each of the first medical device and the second medical device operates an application programming interface to communicate over the network. The first power receptacle may include a first power receptacle communication module that communicates with the first medical device. The second power receptacle may include a second power receptacle communication module that communicates with the second medical device. The first power receptacle communication module may communicate with the second power receptacle communication module. The first power receptacle communication module may wirelessly communicate with the second power receptacle communication module. The first power receptacle communication module may wirelessly communicate with the first medical device. The second power receptacle communication module may wirelessly communicate with the second medical device.

According to a fifth aspect of the disclosed embodiments, a method of providing communication between a first medical device and a second medical device over a localized wireless network includes providing power via a cord from a first power receptacle to the first medical device. The first power receptacle is associated with a wireless communicator to provide wireless communication between the first power receptacle and the first medical device. The first medical device has at least one sensor for acquiring data related to a patient. The method also includes providing power via a cord from a second power receptacle to the second medical device. The second power receptacle is associated with a wireless communicator to provide wireless communication between the second power receptacle and the second medical device. The network is configured such that the first medical device communicates wirelessly with the second medical device. The method also includes publishing the data from the first medical on the network. The method also includes receiving the data at the second medical device from the network.

In some embodiments of the fifth aspect, the method may also include connecting the second medical device to an electronic medical record. The method may also include wirelessly connecting the second medical device to the electronic medical record. The method may also include scanning a patient code to connect the second medical device to the electronic medical record. The electronic medical record may be specifically linked to the patient via the patient code. The patient code may be provided on a patient wristband worn by the patient. The method may also include publishing the data from the second medical device to the electronic medical record.

Optionally, in the fifth aspect, the first medical device may include a patient support apparatus. The second medical device may include at least one of a physiological monitor and an infusion pump. The data may include services provided by the first medical device. The services may include at least one of an electronic medical record channel, a service-oriented device connectivity communication channel, nurse call capabilities, and priority nurse call capabilities. The data may include at least one of patient weight, patient identification, patient blood pressure, patient heart rate, patient respiratory rate, lighting status, caregiver presence indication, and caregiver identification.

It may be desired, in the fifth aspect, that the method also includes operating an application programming interface on each of the first medical device and the second medical device to communicate over the network. The first power receptacle may include a first power receptacle communication module that communicates with the first medical device. The second power receptacle may include a second power receptacle communication module that communicates with the second medical device. The first power receptacle communication module may communicate with the second power receptacle communication module. The first power receptacle communication module may wirelessly communicate with the second power receptacle communication module. The first power receptacle communication module may wirelessly communicate with the first medical device. The second power receptacle communication module may wirelessly communicate with the second medical device.

According to a sixth aspect of the disclosed embodiments, a method of populating an EMR computer with bed status data of a patient bed located in a patient room of a healthcare facility includes establishing a communication channel between a medical device other than the patient bed and the EMR computer. The medical device is located in the patient room with the patient bed. The method also includes communicating the bed status data from the patient bed to a stationary unit located in the patient room with the patient bed. The method also includes communicating the bed status data from the stationary unit to the medical device. The method also includes communicating the bed status data from the medical device to the EMR computer.

In some embodiments of the sixth aspect, the method may also include establishing the communication channel further comprises establishing a long range wireless communication link between the medical device and a wireless access point of a network of the healthcare facility. The long range wireless communication link may include a Wi-Fi communication link. Communicating the bed status data from the patient bed to the stationary unit may also include communicating the bed status data using short range wireless communication technology. The short range wireless communication technology may include Bluetooth communication technology. Communicating the bed status data from the stationary unit to the medical device may also include communicating the bed status data using short range wireless communication technology. The short range wireless communication technology may include Bluetooth communication technology.

Optionally, in the sixth aspect, the medical device may include a physiological monitor. The physiological monitor may include one or more of a heart rate monitor, a respiration monitor, a thermometer, and a blood pressure measuring device. The medical device may include an infusion pump. The medical device may use at least some of the bed status data in connection with operating a function of the medical device. The at least some of the bed status data may include patient weight data as detected by a weigh scale system of the patient bed.

It may be desired, in the sixth aspect, that the method also includes establishing a local mesh network in the patient room between the patient bed, the medical device, and the stationary unit in response to plugging in AC power cords from the patient bed and the medical device into respective AC receptacles of the stationary unit. The method may also include sending a subscribe message from the medical device to the patient bed to subscribe to the bed status data. The subscribe message may include a wireless subscribe message sent directly from the medical device to the patient bed. The subscribe message may be sent to the patient bed via the stationary unit. The patient bed may publish the bed data for receipt by the medical device only after receiving the subscribe message. The stationary unit may be communicatively coupled to a nurse call system that receives the bed status data. The method may also include receiving medical device status data at the stationary unit and communicating the medical device status data to the nurse call system so that the nurse call system receives both the bed status data and the medical device status data.

According to a seventh aspect of the disclosed embodiments, a method includes pairing a patient bed in a patient room with a wall unit in the patient room for wireless communication between the patient bed and the wall unit. The method also includes pairing a medical device in the patient room with the wall unit for wireless communication with the wall unit. The method also includes communicating bed status data from the patient bed to the wall unit. The method also includes communicating the bed status data from the wall unit to the medical device. The method also includes operating a function of the medical device based on the bed status data.

In some embodiments of the seventh aspect, the method may also include communicating the bed status data from the patient bed to the wall unit further comprises communicating the bed status data using short range wireless communication technology. The short range wireless communication technology may include Bluetooth communication technology. Communicating the bed status data from the wall unit to the medical device may include communicating the bed status data using short range wireless communication technology. The short range wireless communication technology may include Bluetooth communication technology.

Optionally, in the seventh aspect, the medical device may include a physiological monitor. The physiological monitor may include one or more of a heart rate monitor, a respiration monitor, a thermometer, and a blood pressure measuring device. The medical device may include an infusion pump. At least some of the bed status data may include patient weight data as detected by a weigh scale system of the patient bed.

It may be desired, in the seventh aspect, that pairing the patient bed in the patient room with the wall unit and pairing the medical device in the patient room with the wall unit may include establishing a local mesh network in the patient room between the patient bed, the medical device, and the wall unit in response to plugging in AC power cords from the patient bed and the medical device into respective AC receptacles of the wall unit. The method may also include sending a subscribe message from the medical device to the patient bed to subscribe to the bed status data. The subscribe message may include a wireless subscribe message sent directly from the medical device to the patient bed. The subscribe message may be sent to the patient bed via the wall unit. The patient bed may publish the bed status data for receipt by the medical device only after receiving the subscribe message. The wall unit may be communicatively coupled to a nurse call system that receives the bed status data. The method may also include receiving medical device status data at the wall unit and communicating the medical device status data to the nurse call system so that the nurse call system receives both the bed status data and the medical device status data.

Additional features, which alone or in combination with any other feature(s), such as those listed above and those listed in the claims, may comprise patentable subject matter and will become apparent to those skilled in the art upon consideration of the following detailed description of various embodiments exemplifying the best mode of carrying out the embodiments as presently perceived.

### BRIEF DESCRIPTION

The detailed description particularly refers to the accompanying figures in which:
Fig. 1 is a schematic diagram of a system for automatically provisioning devices in a localized wireless network, wherein the system includes an outlet and at least one medical device;
Fig. 2 is a front view schematic of the outlet shown in Fig. 1;
Fig. 3 is a front view schematic of the system of Fig. 1 illustrating a first step in a method for provisioning devices in a localized wireless network, wherein the device has not been previously authenticated;
Fig. 4 is a front view schematic of the system of Fig. 1 illustrating a second step in a method for provisioning devices in a localized wireless network, wherein the device has not been previously authenticated;
Fig. 5 is a front view schematic of the system of Fig. 1 illustrating a third step in a method for provisioning devices in a localized wireless network, wherein the device has not been previously authenticated;
Fig. 6 is a front view schematic of the system of Fig. 1 illustrating a fourth step in a method for provisioning devices in a localized wireless network, wherein the device has not been previously authenticated;
Fig. 7 is a front view schematic of the system of Fig. 1 illustrating a fifth step in a method for provisioning devices in a localized wireless network, wherein the device has not been previously authenticated;
Fig. 8 is a front view schematic of the system of Fig. 1 illustrating a method for provisioning devices in a localized wireless network, wherein the device has already been previously authenticated;
Fig. 9 illustrates a localized wireless network having a plurality of outlets and medical devices communicating with each other;
Fig. 10 illustrates a flow chart of a method for provisioning a medical device with an outlet in a localized wireless network;
Fig. 11 illustrates a schematic diagram of a localized wireless network including a first medical device and a second medical device, wherein the first medical device is shown wirelessly connected to an outlet, and the second medical device is being provisioned by the outlet;
Fig. 12 illustrates a schematic diagram of the localized wireless network shown in Fig. 11, wherein the second medical device is configured to receive messages from both the first medical device and the outlet;
Fig. 13 illustrates a schematic diagram of the localized wireless network shown in Fig. 11, wherein the first medical device has published information to the second medical device; and
Fig. 14 illustrates a schematic diagram of the localized wireless network shown in Fig. 11, wherein the second medical device has received the information from the first medical device and published the information to an electronic medical record.

### DETAILED DESCRIPTION

While the concepts of the present disclosure are susceptible to various modifications and alternative forms, specific exemplary embodiments thereof have been shown by way of example in the drawings and will herein be described in detail. It should be understood, however, that there is no intent to limit the concepts of the present disclosure to the particular forms disclosed, but on the contrary, the intention is to cover all modifications, equivalents, and alternatives falling within the spirit and scope of the invention as defined by the appended claims.

Utilizing a time-based method for automatic identification and connection, the disclosed embodiments include a plurality of smart outlets that are associated with one another in a medical environment (i.e. a patient room in a healthcare facility). The smart outlets described herein automatically provision devices plugged into the smart outlet with the localized network with which the outlet is associated. The net effect is that any medical device plugged into the smart outlet will be automatically associated with a localized, wireless network and can communicate on that network with other devices. In some embodiments, the localized wireless network is a Bluetooth Mesh network. It will be appreciated that, in some embodiments, the smart outlets described herein are usable with a network in a non-healthcare environment to connect non-medical devices.

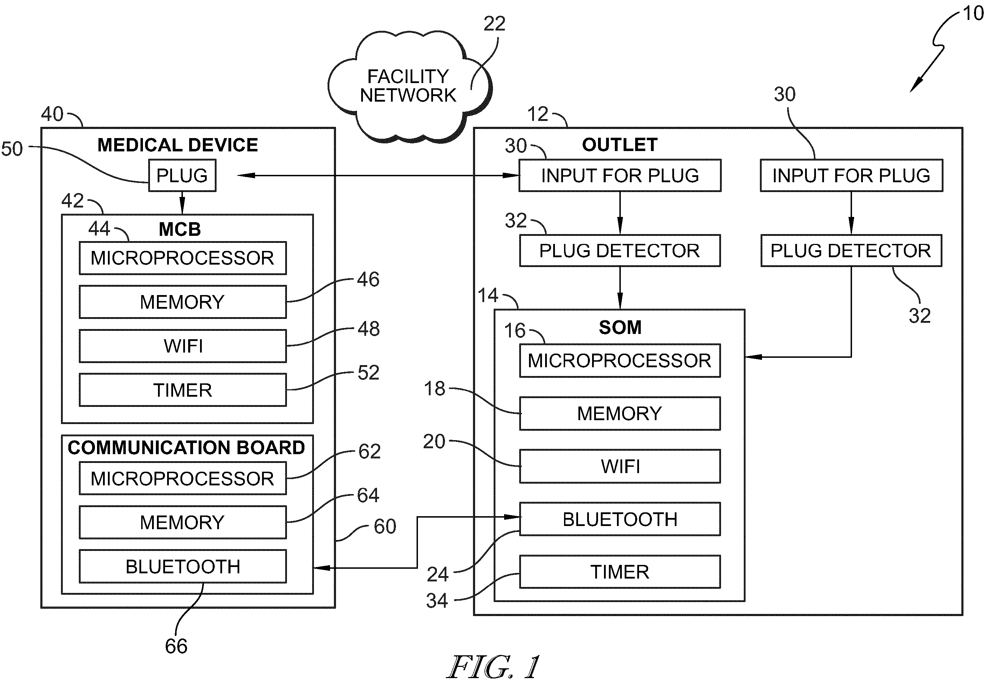

Referring now to Fig. 1, a system 10 for automatically provisioning devices in a localized wireless network includes at least one smart outlet 12. The smart outlet 12 is sometimes referred to herein as a wall module 12, a wall unit 12, a stationary unit 12, or just module 12 or unit 12, or terms of similar import. Thus, the present disclosure contemplates that outlets 12 can be mounted directly to a room wall of a building or can be mounted to some other piece of architectural equipment such as a service chase in a room; a service column attached to a room wall, ceiling or floor; an equipment support arm fixed to a room wall or room ceiling; a headwall unit; a bed locator unit; and like structures that are permanently provided in a healthcare facility as part of the infrastructure of healthcare facility, for example. If attached to a service column that, in turn, is mounted to a wall or ceiling by a movable arm assembly, outlet 12 is still considered to be a stationary unit 12 or wall module 12, etc., because it is affixed to a panel (e.g., wall) or frame member of the column and remains in the corresponding room on an ongoing basis.

Smart outlet 12 includes a circuit board 14. In the illustrated embodiment, the circuit board 14 is a system-on-module (SOM) circuit board. The circuit board 14 includes an outlet processor 16 configured to carry out instructions stored in an outlet memory 18. A wireless module 20 enables the outlet processor 16 to communicate with and share data with a facility network 22. An outlet communication module 24 enables the outlet processor 16 to communicate with and share data with other devices, as described in more detail below. In some embodiments, the outlet communication module 24 is a Bluetooth module. The smart outlet 12 includes at least one power receptacle 30 for a plug and a plug detector 32 associated with each power receptacle 30. Although the illustrated embodiment shows two power receptacles 30, it will be appreciated that the smart outlet 12 includes any number of power receptacles 30 and associated plug detectors 32, in some embodiments. Each of the power receptacles 30 is in communication with the circuit board 14. In some embodiments, the power receptacles 30 include an alternating current power receptacle. The plug detectors 32 detect a plug inserted into the associated power receptacle 30 and transmits a signal to the outlet processor 16 to begin a provisioning routine, as described in more detail below. A timer 34 is started to measure an uptime in response to a power plug being plugged into a power receptacle 30 as detected by the plug detector 32.

The system 10 is configured to provision at least one medical device 40. The medical device 40 includes a circuit board 42. In the illustrated embodiment, the circuit board 42 is a master control board (MCB) board. The circuit board 42 includes a device processor 44 that carries out instructions stored in a memory 46. A wireless module 48 enables the device processor 44 to communicate with and share data with the facility network 22. A timer 52 measures an uptime in response to sensing that the medical device 40 is receiving power. A power plug 50 is configured to insert into one of the power receptacles 30 of the smart outlet 12. A communication circuit board 60 includes a communication processor 62 that carries out instructions stored in a memory 64. The circuit board 60 includes a device communication module 66 that is configured to communicate with the outlet communication module 24 so that the medical device 40 and the smart outlet 12 can share data. In some embodiments, the device communication module 66 is a Bluetooth module.

As described in more detail below, the plug detector 32 detects the power plug 50 inserted into the power receptacle 30 and transmits a signal to the outlet processor 16 to begin a provisioning routine. During the provisioning routine, the outlet processor 16 of the smart outlet 12 authenticates the medical device 40 and connects the medical device 40 to the localized network. In the localized network, the medical device 40 is enabled to communicate and exchange data with the smart outlet 12 or any other devices provisioned to the localized network. In some embodiments, the plug detector 32 also recognizes removal of the power plug 50, and upon removal of the power plug 50, the medical device 40 is disconnected from the localized network. In some embodiments where the medical device 40 includes a battery, the medical device 40 remains connected to the localized network after removal of the power plug 50 until the medical device 40 is manually disconnected from the localized network or moved out of range from the localized network.

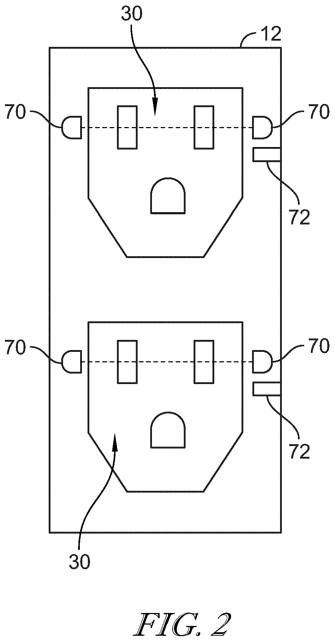

Fig. 2 illustrates a smart outlet 12 having two power receptacles 30. It will be appreciated that the smart outlet 12 has any number of power receptacles 30, in some embodiments. In the illustrated embodiment, the power receptacles 30 are three prong power receptacles. In other embodiments, the power receptacles 30 are any suitable type of power receptacle. In the exemplary embodiments, the plug detector 32 of the outlet 12 includes infrared beam transceivers 70 that cooperate to detect the presence of at least one prong of the power plug 50 of the medical device 40 being inserted into the power receptacle 30. For example, one infrared beam transceiver 70 emits infrared (IR) light in a generally horizontal direction for detection by the other infrared beam transceiver 70 and the at least one prong blocks the IR light from reaching the other infrared beam transceiver 70 after the power plug is plugged into the power receptacle 30.

In some embodiments, the plug detector 32 includes a mechanical switch that moves from a first state to a second state in response to the power plug 50 of the medical device 40 being plugged into the power receptacle 30. For example, in some embodiments, the mechanical switch includes a plunger switch having a plunger that is pressed inwardly by a plug body of the power plug 50 when the power plug 50 is plugged into the power receptacle 30. In other embodiments, the plug detector 32 includes a current sensor to sense current flowing to at least one prong of the power plug 50 after the power plug 50 is plugged into the power receptacle 30. In some embodiments, the plug detector 32 includes a reader that detects a tag coupled to the power plug 50. The tag carries a transponder that is read by the reader. For example, the transponder, in some embodiments, includes a near field communication (NFC) transponder. The NFC transponder is included in an NFC integrated circuit chip, in some embodiments. Optionally, the reader may emit energy to power the transponder to enable the transponder to send a signal back to the reader.

Each power receptacle 30 also includes an indicator 72 configured to represent a status of the power receptacle 30. In some embodiments, the indicator 72 includes a multi-colored light, for example, a multi-colored light emitting diode. In some embodiments, the status of the power receptacle 30 includes a connection status of the outlet communication module 24. For example, the indicator 72 is illuminated in a first color when the outlet communication module 24 is not connected to the localized network, and the indicator 72 is illuminated in a second color when the outlet communication module 24 is connected to the localized network. In some embodiments, the indicator 72 is illuminated in a first color when a power plug 50 is not detected by the plug detector 32, and the indicator 72 is illuminated in a second color when a power plug 50 is detected by the plug detector 32. In some embodiments, the outlet processor 16 updates the indicator 72 based on the status of the power receptacle 30.

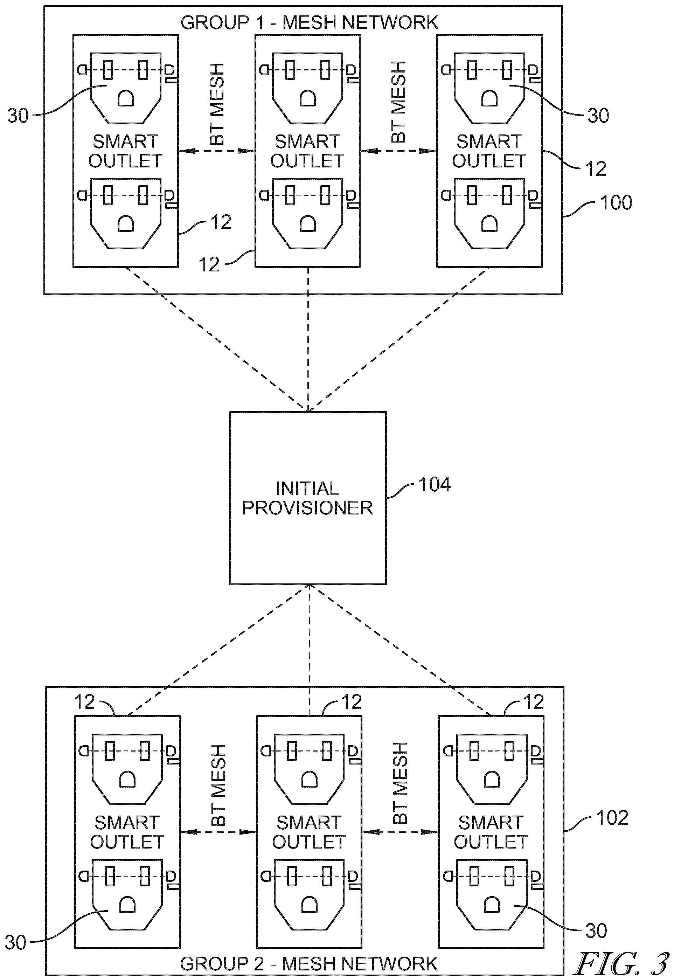

Fig. 3 illustrates a first group 100 of smart outlets 12 and a second group 102 of smart outlets 12. The groups 100 and 102 are each provided in a healthcare facility. It will be appreciated that, in other embodiments, the groups 100 and 102 are provided in non-healthcare related facilities. In some embodiments, the groups 100 and 102 are provided in the same room of the healthcare facility, for example, a patient room. Initially, the smart outlets 12 of each group 100 and 102 are not in communication with each other.

An initial provisioner 104 is utilized to establish communication between the smart outlets 12 of the groups 100 and 102. The initial provisioner 104 is a tablet or handheld device, in one embodiment. The initial provisioner 104 is equipped with a Bluetooth connection and individually connects to each smart outlet 12. Each group 100 and 102 is then assigned a unique GroupID. Each smart outlet 12 that belongs to that group 100 or 102 is provisioned and assigned that GroupID by the initial provisioner 104. A Bluetooth mesh is established between the smart outlets 12 of each respective group 100 and 102. Accordingly, each smart outlet 12 is capable of provisioning new devices for its respective group 100 or 102 using the localized network.

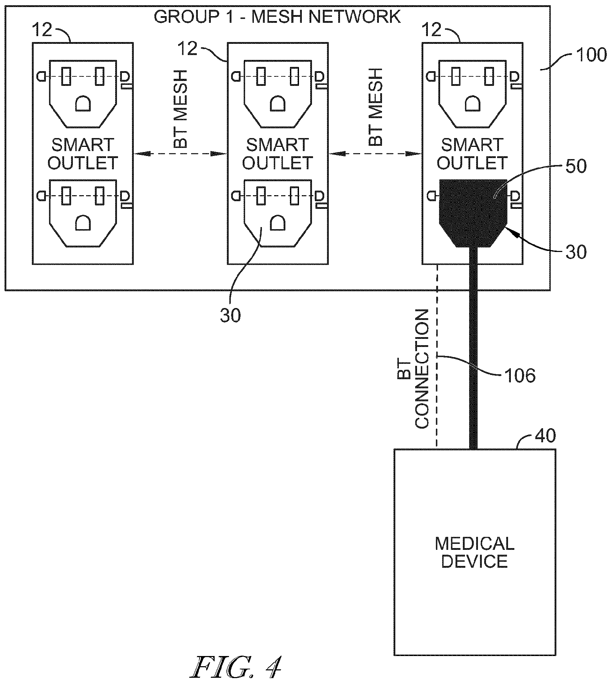

Referring to Fig. 4, when a device 40, for example a medical device, wants to join the localized network, the device 40 is plugged into a power receptacle 30 of a smart outlet 12 in the group 100 the device 40 is intended to join. Using the system and methods described in U.S. Patent Serial No. 17/577,496, published as U.S. Patent Application Publication No. 2022/0233382 A1, which is incorporated in its entirety herein by reference, the smart outlet 12 and device 40 make a secured, one-to-one Bluetooth connection 106. This step establishes an automatic, secure wireless connection between the associated smart outlet 12 and the device 40 over which the smart outlet 12 and the device 40 can exchange data. In some embodiments, for devices 40 that do not have a Bluetooth Mesh protocol stack, the smart outlets 12 include a proxy node to allow the device 40 to still interact with the mesh network through a direct low energy connection to the smart outlet 12 using a proxy protocol.

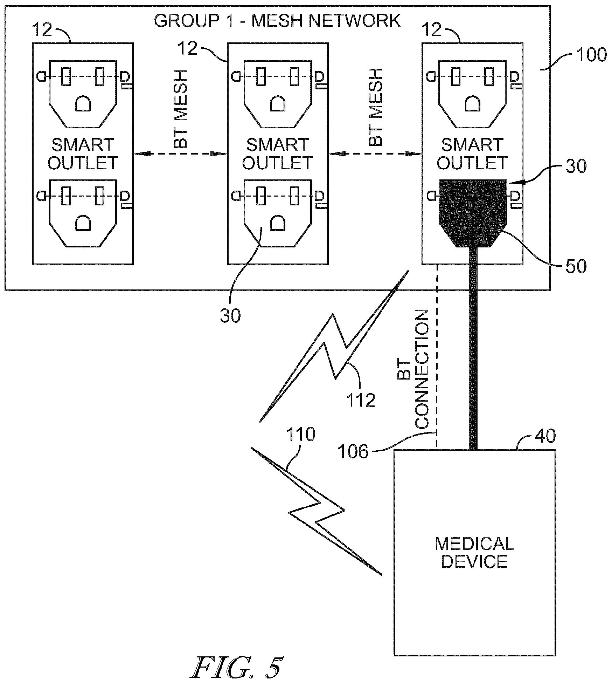

Referring to Fig. 5, the device 40 then performs a beaconing step 110 and the smart outlet 12 sends a provisioning invite protocol data unit 112 over an unsecured link. That is, the device 40 broadcasts its availability to be provisioned, and the associated smart outlet 12 creates a second, unsecured link to the device 40 and sends a provisioning invite 112. Once the link is established, the device 40 and the smart outlet 12 exchange public keys. In some embodiments, this exchange is done either with the secure, one-to-one connection or over the second, unsecured link.

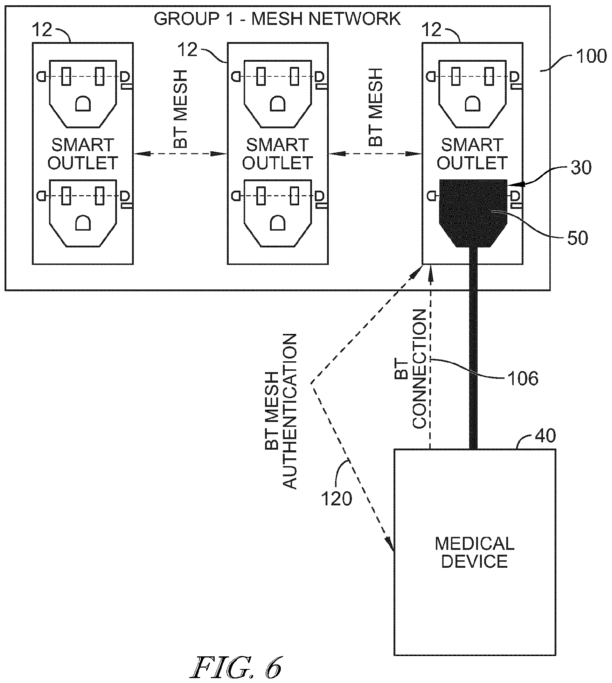

Referring to Fig. 6, the device 40 or the smart outlet 12 then generates a random number 120 to be used for a cryptographic exchange. That is, in order to generate secure, cryptographic material, a shared secret must be obtained. In the disclosed embodiments, the secret number 120 is generated by the device 40 and shared with the associated smart outlet 12 over the secure one-to-one connection. Alternatively, the associated smart outlet 12 generates the number and sends it to the device 40. In the disclosed embodiments, the number 120 is securely transmitted without user intervention. Once received the smart outlet 12 and device 40 authenticate to one another by exchanging confirmation values and verifying those values.

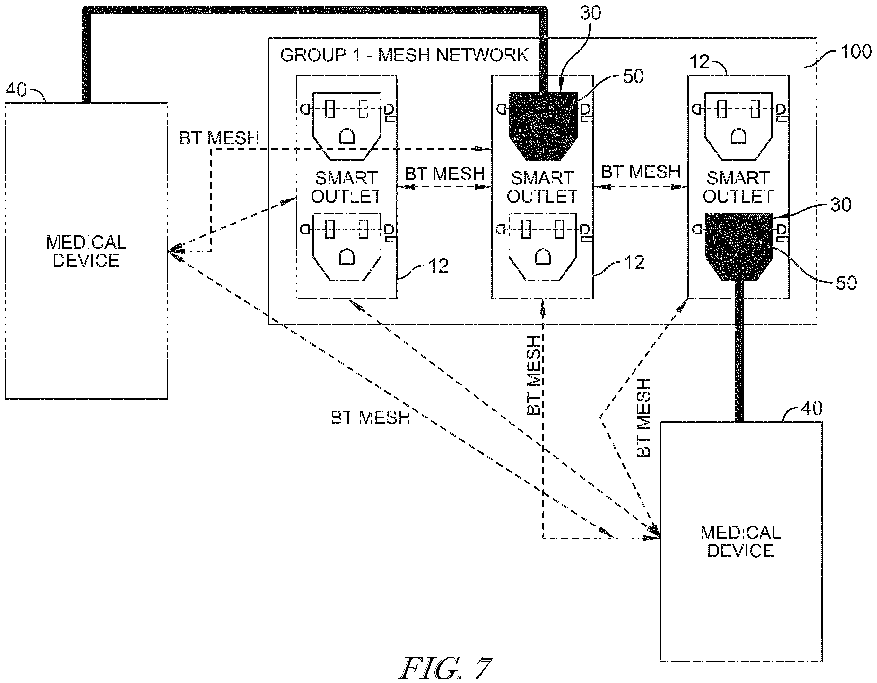

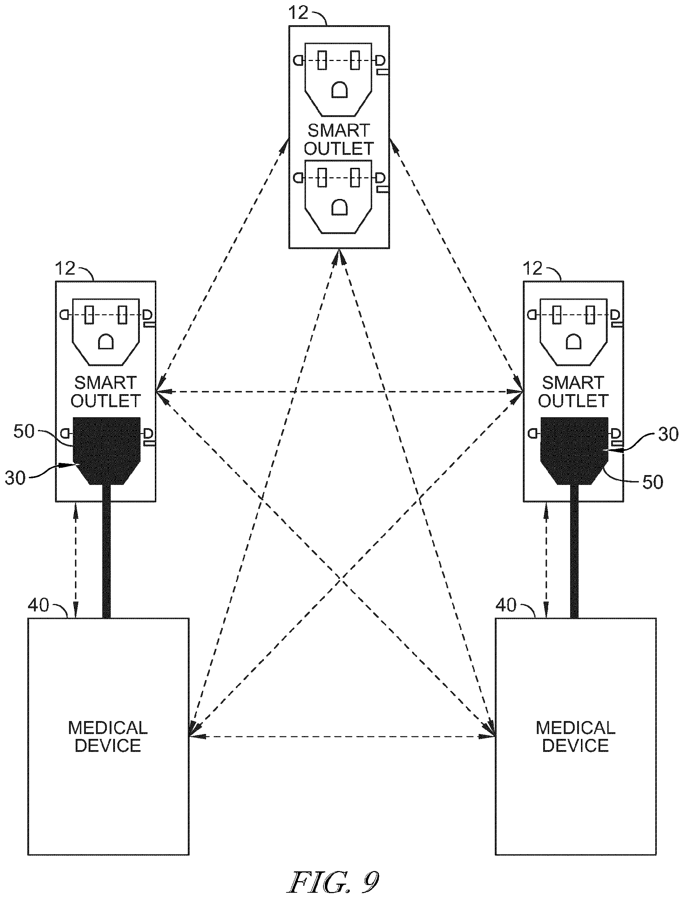

Referring now to Fig. 7, provisioning data is then sent to the device 40 including a mesh network netkey. The associated smart outlet 12 generates the provisioning data and sends the data to the device 40 over the newly secured link. Once the device 40 receives this provisioning data, the device 40 becomes part of the mesh network and is capable of publishing and subscribing to models on the network from other devices 40 and other smart outlets 12 in the same group 100. The one-to-one connection with the outlet 12 can then be dropped, if desired. In some embodiments, the connection to the smart outlet 12 is maintained if that specific outlet has additional features that the device 40 can use. The Bluetooth mesh network allows for any device 40 in the network to exchange data with another device 40 on the same network, as shown in Fig. 9. As illustrated in Fig. 9, two devices 40 are not required to be plugged into the same outlet 12 to communicate with one another over the network. Rather, a device 40 plugged into a first outlet 12 can communicate with another device 40 plugged into a second outlet 12, when the first outlet 12 and the second outlet 12 are on the same network, as illustrated in Fig. 9. The Bluetooth Mesh uses a publish/subscribe model, in one embodiment. It will be appreciated that a model on the network can be a client, server or control model. In some embodiments, groups of smart outlets 12 are configured to communicate over the localized wireless network. For example, in some embodiments, the smart outlets 12 of group 100 and the smart outlets 12 of the group 102 are each provisioned to the same localized wireless network and communicate over the same localized wireless network.

Accordingly, in an exemplary embodiment, the medical device 40 determines that the medical device 40 is receiving power via the power plug 50 and the power receptacle 30. The smart outlet 12 also detects the power plug 50 being plugged into the power receptacle 30 via the plug detector 32. The timer 52 of the medical device 40 starts to measure a first uptime in response to sensing that the medical device 40 is receiving power via the power plug 50 and the power receptacle 30. The timer 34 of the smart outlet 12 starts to measure a second uptime in response to the power plug 50 being plugged into the power receptacle 30. The medical device 40 then transmits to the smart outlet 12 from the wireless module 48 an advertisement including the first uptime. The smart outlet 12 compares the first uptime with the second uptime and, if the first uptime is within a predetermined tolerance range of the second uptime, the smart outlet 12 sends the pairing message to the medical device 40 which results in the smart outlet 12 and the medical device 40 becoming automatically paired for subsequent wireless communications. The medical device 40 is then configured to send wireless messages to, and receive wireless messages from, at least one wireless access point of the network. Additionally, the smart outlet 12 is then configured to send wireless messages to, and receive wireless messages from, the at least one wireless access point of the network.

In some embodiments, the medical device 40 is configured to transmit a device identification (ID) to the smart outlet 12 and the smart outlet 12 is configured to transmit the device ID and a location ID to at least one server of the network 22 of the healthcare facility. The location ID is correlatable to a location at which the medical device 40 is located in the healthcare facility. In some embodiments, the medical device 40 includes a graphical display screen and the smart outlet 12 is configured to transmit from the wireless module 20 to the wireless module 48 of the medical device 40 a smart text string that may be displayed on the graphical display screen. The smart text string includes a name of the location at which the medical device 40 is located and may be different than the location ID.

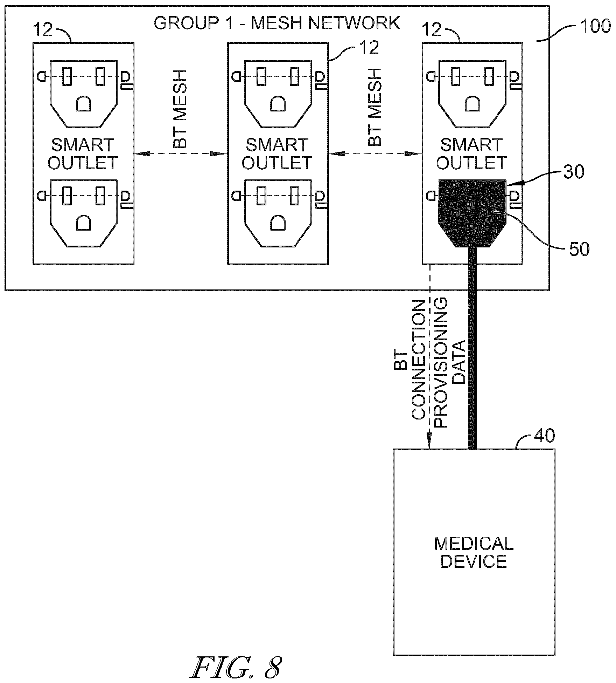

In another embodiment, shown in Fig. 8, wherein the device 40 and the smart outlet 12 had previously exchanged public keys and established a Bluetooth connection, the associated smart outlet 12 generates the provisioning data and sends the data to the device 40 over the secure one-to-one connection. Once the medical device receives this provisioning data, the device 40 is now a member of the mesh network and able to communicate with other devices 40 on the network. The traditional Bluetooth mesh provisioning steps are designed to facilitate the secure exchange of the provisioning data. All but the last step in the provisioning process are designed to build a secure communication link for that exchange. In this embodiment, a secure communication link already exists in the form of the secure, one-to-one connection made using the time-based pairing method already disclosed. This link can be used to exchange sensitive information without risk of interception or manipulation. This embodiment reduces steps and improves efficiency.

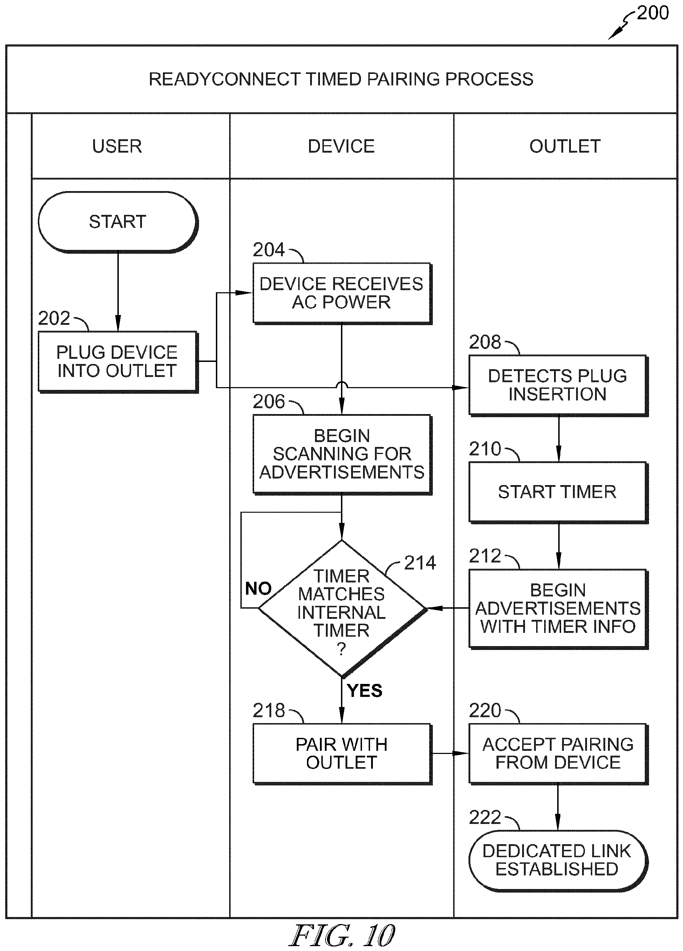

Referring now to Fig. 10, a flow chart 200 for connecting a device 40 to a smart outlet 12 is provided. At step 202, as user plugs the power plug 50 of the device 40 is plugged into the power receptacle 30 of a smart outlet 12. At step 204, the device 40 receives power from the smart outlet 12, and at step 206, the device 40 begins scanning for advertisements. Nearly simultaneously, at step 208, the smart outlet 12 detects the device 40 and starts a timer, at step 210. At step 212, the smart outlet 12 sends advertisements with the timer info. At step 214, the device processor 44 determines whether the timer info from the smart outlet 12 matches the internal timer. If the timer info from the smart outlet 12 does not match the internal timer, the device 40 continues to scan for advertisements, at step 206. If the timer info from the smart outlet 12 matches the internal timer, the device 40 pairs with smart outlet 12, at step 218. At step 220, the smart outlet 12 accepts the pairing and a dedicated link is established, at step 222.

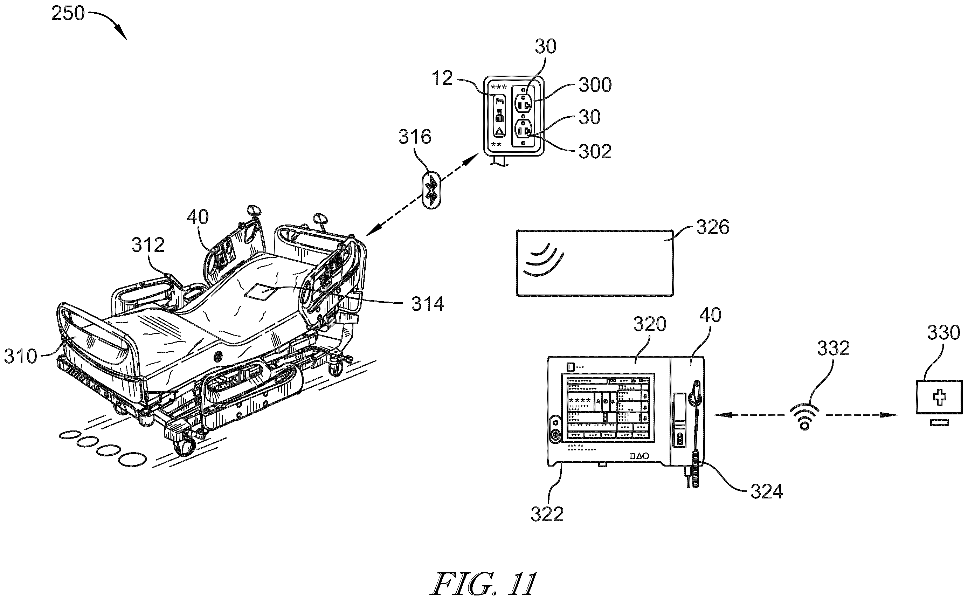

Referring now to Figs. 11-14, a system or localized wireless network 250 is illustrated for use in a healthcare facility. It will be appreciated that, while medical devices 40 and wall unit 12 are provided in a common patient room, the overall system 250 includes other computer devices that may extend across multiple rooms or locations of the facility. In some embodiments, the network 250 includes an in-room Bluetooth Mesh network or other localized network using short-range (e.g., about 3 feet (about 1 meter) to about 20 feet (about 6 meters)) communication technology. The network 250 includes at least one of the smart outlets 12 of the type discussed hereinabove having two power receptacles 30 embodied as a first power receptacle 300 and a second power receptacle 302. The outlet 12 includes the communication module 24.

In some embodiments, the network 250 includes more than one outlet 12 in the same room, wherein each outlet 12 includes a communication module 24 (as shown in Fig. 1). In such embodiments, the communication module 24 of each outlet 12 communicates over the network 250 with the communication modules 24 of the other outlets 12 in the room. For example, the communication module 24 of a first outlet having the first power receptacle 300 communicates with the communication module 24 of a second outlet having the second power receptacle 302. That is, it is contemplated that receptacles 300, 302 are provided on separate wall modules 12 instead of being on the same module 12 as shown in the example of Figs. 11-14. In some embodiments, the communication modules 24 communicate wirelessly, although this is not to exclude the possibility that wired connections between communication modules 24 of wall units 12 can be provided, if desired. In any event, in some embodiments, the communication modules 24 communicate with each other using Bluetooth technology.

In the illustrative example of Figs. 11-14, one medical device 40 is embodied as a first medical device 310 in the form of a patient support apparatus 312 plugged into the first power receptacle 300. As illustrated in Fig. 11, the first medical device 310 has already been provisioned onto the network 250 using one of the methods described above to pair the first medical device 310 to the wall unit 12. The first medical device 310 includes a communication module 66 (shown in Fig. 1). In some embodiments, the communication module 66 operates an application programming interface to communicate over the network 250. The communication module 24 of the outlet 12 communicates with the communication module 66 of the first medical device 310. In some embodiments, the communication module 24 of the outlet 12 communicates with the communication module 66 of the first medical device 310 over a short-range wireless communication link 316. In some embodiments, the wireless communication link 316 is a Bluetooth communication link.

The first medical device 310 includes at least one sensor 314 for acquiring data related to a patient positioned on the first medical device 310. In an exemplary embodiment, the data includes bed status data. It should be appreciated that the at least one sensor 314 is depicted diagrammatically as a single block in Figs. 11-14 in connection with patient support apparatus 312 but that patient support apparatuses 312 having multiple sensors 314 at various locations of the respective patient support apparatuses 312 are within the scope of this disclosure. For example, the at least one sensor 314 includes multiple load cells (e.g., four load cells) that are included in a weigh scale system of the respective patient support apparatus 312 in some embodiments. Accordingly, the data acquired by such load cells includes a patient weight. Alternatively or additionally, the at least one sensor 314 includes a blood pressure cuff and the acquired data includes the patient's blood pressure. Further alternatively or additionally, the at least one sensor 314 includes a sensor of a heart rate monitor and the acquired data includes the patient's heart rate. Still further alternatively or additionally, the at least one sensor 314 includes a sensor of a respiratory rate monitor and the acquired data includes the patient's respiratory rate. The same sensor 314 used to acquire the patient's heart rate is also used to acquire the patient's respiration rate in some embodiments.

In some embodiments, a patient identification number (aka patient ID) is entered into memory of one of medical devices 40 and is communicated to one or more other medical devices 40 or to other devices (e.g., module 12 or other computer devices of system 250) for subsequent transmission with the acquired data of the respective medical device 40. For example, the patient ID is entered into memory of the respective device 40 by scanning a wrist band of the patient in some embodiments, or is transmitted to one of devices 40 from an EMR system computer or an ADT system computer, for example. Embodiments in which the patient ID is entered using an input device (e.g., keyboard, touchscreen, etc.) of the respective medical device 40 is also contemplated by the present disclosure. Alternatively or additionally, the patient ID is entered into the memory of a respective medical device 40 via a tag reader of the medical device detecting a wireless signal emitted by an ID tag worn by the patient. Caregiver ID's also may be detected by the medical device 40 in a similar manner based on wireless communication with ID tags worn by caregivers. In some embodiments, one of devices 40 has stored in the memory thereof other data such as one or more of a status of a lighting system in the patient room, a status of a caregiver presence indicator and/or a caregiver ID of a detected caregiver or a caregiver that is otherwise assigned for caring for the patient.

In some embodiments, the first medical device 310 includes various services for the patient and the caregiver. For example, the services include one or more of the following: an electronic medical record channel, a service-oriented device connectivity (SDC) communication channel, and/or nurse call capabilities including priority nurse call capabilities, if desired. In the embodiment of Figs. 11-14, the services of the first medical device 310 are capable of being published over the network 250 such as to other medical devices 40, for example.

With continued reference to Figs. 11-14, one of the medical devices 40 is embodied as a second medical device 320 that is plugged into the second power receptacle 302. In some embodiments, the second medical device 320 includes a physiological monitor. In some embodiments, the physiological monitor includes one or more of a heart rate monitor, a respiration monitor, a thermometer, and a blood pressure measuring device. In some embodiments, the second medical device 320 includes an infusion pump. The second medical device 320 includes a communication module 66 (shown in Fig. 1) that operates an application programming interface to communicate over the network 250. In the illustrative example, the second medical device 320 includes a scanner 324 configured to scan a patient code on a wristband worn by the patient. For example, scanner 324 includes a bar code reader or QR code reader, or other camera-based reader to read visual indicia. As illustrated in Fig. 11, after medical device 320 is moved into the patient room in which patient support apparatus 310 is located, the corresponding outlet 12 transmits a group identifier 326 to the second medical device 320 to provision the second medical device 320 to the network 250 using one of the methods described above.

An electronic medical record (EMR) computer 330, such as an EMR server of an EMR system, is communicatively coupled to the second medical device 320 as shown diagrammatically in Figs. 11-14. In the illustrated embodiment, the electronic medical record computer 330 is connected to second medical device 320 via a long range wireless communication link 332 between the second medical device 320 and a wireless access point of the network 22 of the healthcare facility. In some embodiments, the long range wireless communication link includes a Wi-Fi communication link for longer range communication than the Bluetooth communications connections between medical devices 40 and wall unit 12. The scanner 324 is configured to scan the patient code to connect to the patient's electronic medical which is stored in the electronic medical record computer 330. That is, the patient's electronic medical record is specifically linked to the patient via the patient code in computer 330. For example, the electronic medical record in computer 330 is linked to the patient with a patient ID number embedded in the patient code that is read by the scanner 324.

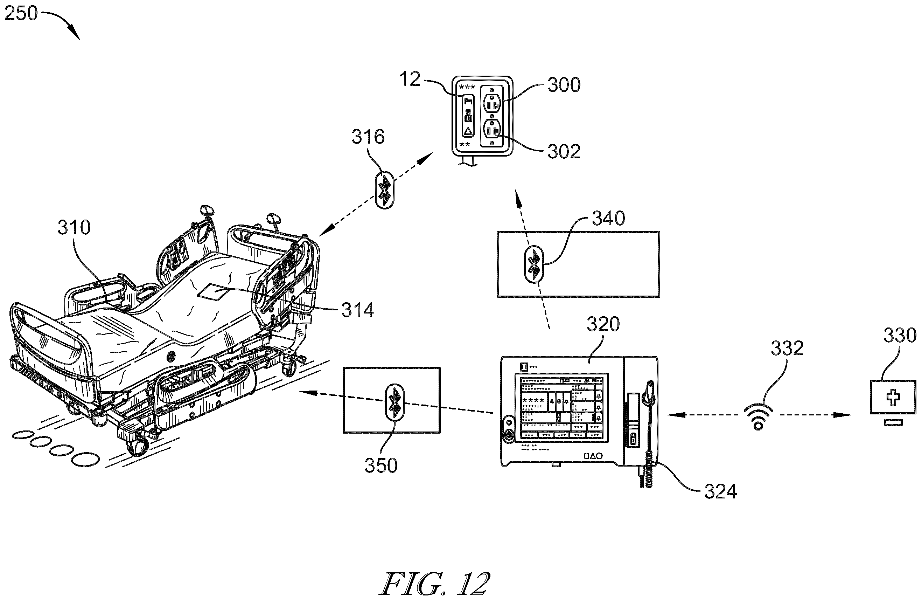

Referring to Fig. 12, the second medical device 320 is provisioned to the network 250 using one of the methods described above to pair the second medical device 320 with the wall unit 12 so that the communication module 24 of the second power receptacle 302 communicates with the communication module 66 of the second medical device 320 over the network 250. In some embodiments, the communication module 24 of the second power receptacle 302 communicates with the communication module 66 of the second medical device 320 over a short-range wireless communication link 340. In some embodiments, the wireless communication link 340 is Bluetooth communication link. This enables the communication module 66 of the first medical device 310 to pair with the second medical device 320 and communicate with the communication module 66 of the second medical device 320 across a short-range wireless communication link 350. In some embodiments, the wireless communication link 350 is a Bluetooth communication link.

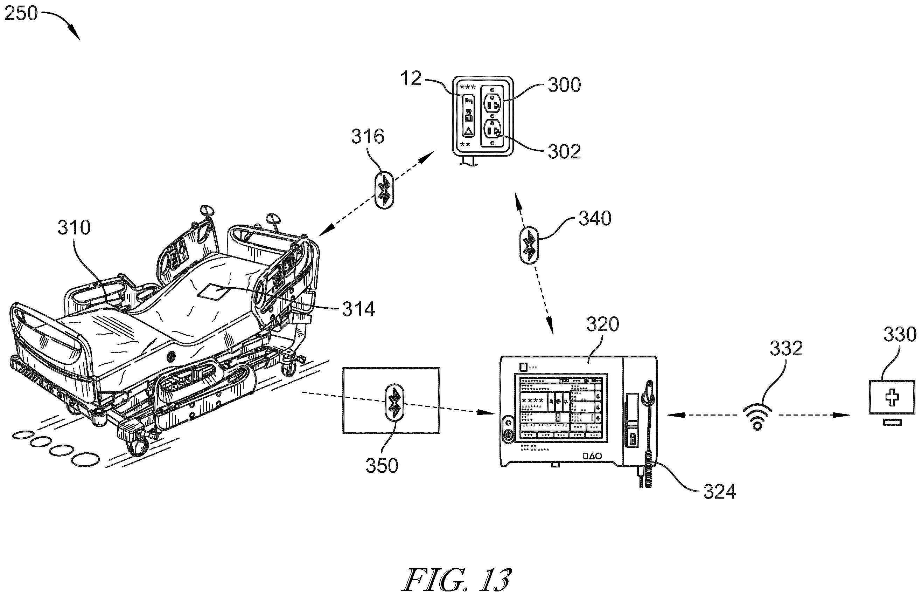

Accordingly, as illustrated in Fig. 13, a communication channel is established between the first medical device 310, the second medical device 320, and the EMR computer 330. This communication channel can include a first communication path including communication links 316, 340, 332 or a second communication path including communication links 350, 332 or both sets of communication links for redundancy of transmitted data, if desired. In some embodiments, the second medical device 320 sends a subscribe message to the first medical device 310 to subscribe to the data. In some embodiments, the subscribe message includes a wireless subscribe message sent directly from the second medical device 320 to the first medical device 310. In some embodiments, the subscribe message is sent to the first medical device 310 via the wall unit 12. As such, the first medical device 310 is capable of publishing information to the second medical device 320. In some embodiments, the first medical device 310 publishes the data for receipt by the second medical device 320 only after receiving the subscribe message. In some embodiments, the first medical device 310 publishes the information to the second medical device 320 across the wireless connection 350. In an exemplary embodiment, the information includes the data acquired by the sensor 314 and the services of the first medical device 310. Based on the foregoing, therefore, the following data objects for publishing within system 250 between devices 40 include, but are not limited to, patient weight, patient ID, blood pressure, EMR communication channel (e.g., the ability to communicate with the EMR computer 330), SDC communication channel (e.g., the ability to communicate according to the IEEE 11073 service-oriented device connectivity (SDC) family of standards that defines a communication protocol for point-of-care (PoC) medical devices), caregiver presence indication, nurse call system communication capability including priority nurse call capability, patient heart rate and/or respiration rate, alert light status (e.g., SafeView light status of the Centrella Smart+ bed available from Hill-Rom Company, Inc. of Batesville, Indiana), and caregiver ID.

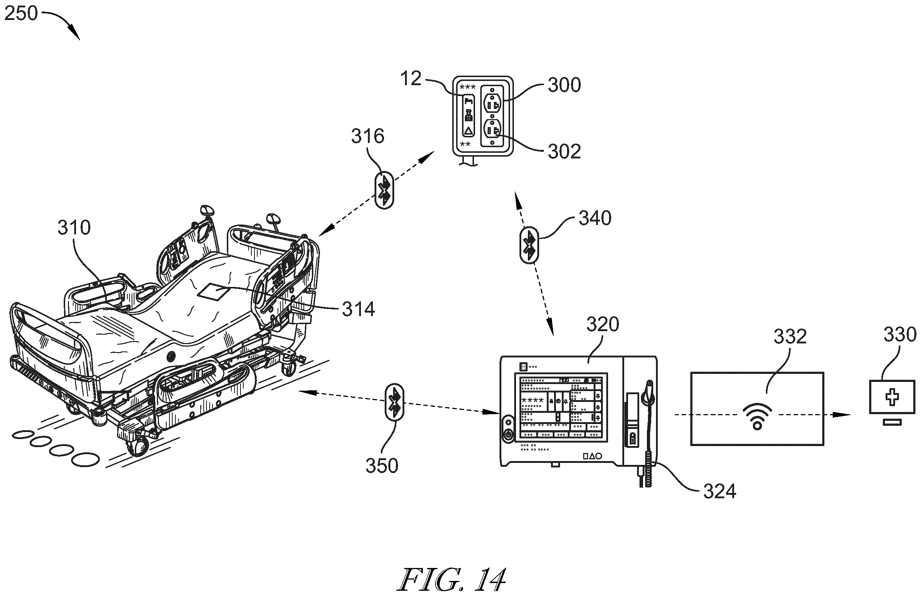

As shown in Fig. 14, the second medical device 320 receives the information from the first medical device 310. Accordingly, the second medical device 320 is capable of utilizing the data and the services received from the first medical device 310. Additionally, the second medical device 320 is capable of publishing the information to the electronic medical record computer 330 over the wireless connection 322. In some embodiments, the wall unit 12 is communicatively coupled to a nurse call system that receives the data related to the first medical device 310 (bed status data). In some embodiments, the wall unit 12 also receives data related to the second medical device 320 (medical device status data). In such embodiments, the wall unit 12 communicates the medical device status data to the nurse call system so that the nurse call system receives both the bed status data and the medical device status data. In some embodiments, including the illustrative embodiment of Figs. 11-14, second medical device 320 comprises a CONNEX^{®} Spot Monitor (CSM) available from Welch Allyn, Inc. of Skaneateles Falls, New York.

Embodiments of the invention can be described with reference to the following numbered clauses:

1. A system for automatically provisioning devices in a localized wireless network, the system comprising:
a plurality of power receptacles, wherein each power receptacle includes a plug detector in communication with an outlet processor of an outlet, and
an outlet communication module in communication with the processor,
wherein each power receptacle is configured to provision an electronic device, wherein the electronic device has:
   a device processor, and
   a device communication module in communication with the device processor,
   wherein, in response to a first power plug of a first electronic device being inserted into a first power receptacle of the plurality of power receptacles and detected by a first plug detector associated with the first power receptacle, the outlet processor instructs the outlet communication module to communicate with the device communication module of first electronic device to add the first electronic device to a localized wireless network,
   wherein, in response to a second power plug of a second electronic device being inserted into a second power receptacle of the plurality of power receptacles and detected by a second plug detector associated with the second power receptacle, the outlet processor instructs the outlet communication module to communicate with the device communication module of second electronic device to add the second electronic device to the localized wireless network, and
   wherein the device communication module of the first electronic device is enabled to communicate with the device communication module of the second electronic device through the localized wireless network.
2. The system of clause 1, wherein each power receptacle includes an indicator configured to represent a status of the power receptacle.
3. The system of clause 2, wherein the indicator includes a multi-colored light.
4. The system of clause 2 or 3, wherein the status includes a connection status of the outlet communication module.
5. The system of any one of clause 2 to 4, wherein the outlet processor is configured to update the indicator based on the status of the power receptacle.
6. The system of any preceding clause, wherein the outlet communication module is wireless.
7. The system of clause 6, wherein the outlet communication module communicates with the first device communication module and the second device communication module wirelessly.
8. The system of any preceding clause, wherein the outlet processor is configured to detect the insertion of a power plug into one of the plurality of power receptacles with the associated plug detector.
9. The system of any preceding clause, wherein the outlet processor is configured to exchange data with the first electronic device and the second electronic device.
10. The system of any preceding clause, wherein the device processor of each of the plurality of electronic devices is configured to exchange data with the outlet processor over the localized wireless network.
11. The system of clause 10, wherein the device processor of each of the first electronic device and the second electronic device is configured to exchange data with the device processor of other electronic device of the plurality of electronic devices over the localized wireless network.
12. The system of any preceding clause, wherein the outlet establishes communication with each of the first electronic device and the second electronic device by generating and sending provisioning data, in response to the respective power plug being inserted into one of the plurality of power receptacles and detected by the associated plug detector.
13. The system of any preceding clause, wherein each of the first electronic device and the second electronic device establishes communication with the outlet by sending a signal to the outlet processor indicating an availability to connect, in response to the respective power plug being inserted into one of the plurality of power receptacles and detected by the associated plug detector.
14. The system of clause 13, wherein the outlet generates and sends a provisioning invite to the respective electronic device, in response to receiving the signal from the respective electronic device.
15. The system of clause 14, wherein the outlet and the respective electronic device exchange public keys, in response to the respective electronic device receiving the provisioning invite.
16. The system of clause 15, wherein a cryptographic exchange occurs between the outlet and the respective electronic device, in response to the exchange of public keys.
17. The system of clause 16, wherein the outlet generates and sends provisioning data to the respective electronic device, in response to the cryptographic exchange.
18. The system of any preceding clause, wherein each of the first electronic device and the second electronic device includes a medical device.
19. The system of any preceding clause, wherein the outlet includes a power outlet in a healthcare facility.
20. The system of any preceding clause, wherein each power receptacle includes an alternating current power receptacle.
21. A method for automatically provisioning devices in a localized wireless network, the method comprising:
   creating a localized wireless network between a plurality of power receptacles, wherein each power receptacle is associated with an outlet communication module, wherein creating the localized wireless network includes generating communication between the plurality of power receptacles,
   detecting the insertion of a first power plug of a first electronic device into a first power receptacle of the plurality of power receptacles,
   generating communication between the outlet communication module and the first electronic device to add the first electronic device to the localized wireless network,
   detecting the insertion of a second power plug of a second electronic device into a second power receptacle of the plurality of power receptacles,
   generating communication between the outlet communication module and the second electronic device to add the second electronic device to the localized wireless network, and
   providing communication between the first electronic device and the second electronic device through the localized wireless network.
22. The method of clause 21, further comprising establishing communication between the outlet communication module and each of the first electronic device and the second electronic device by generating and sending provisioning data from the outlet communication module to the first electronic device and the second electronic device, in response to the respective power plug being inserted into one of the plurality of power receptacles.
23. The method of clause 21 or 22, further comprising establishing communication between the localized wireless network and each of the first electronic device and the second electronic device by sending a signal from the respective electronic device to the outlet communication module indicating an availability to connect, in response to the respective power plug being inserted into one of the plurality of power receptacles.
24. The method of clause 23, further comprising generating and sending a provisioning invite from the outlet communication module to the respective electronic device, in response to receiving the signal from the respective electronic device.
25. The method of clause 24, further comprising exchanging public keys between the outlet communication module and the respective electronic device, in response to the respective electronic device receiving the provisioning invite.
26. The method of clause 25, further comprising generating a cryptographic exchange between the outlet communication module and the respective electronic device, in response to the exchange of public keys.
27. The method of clause 26, further comprising generating and sending provisioning data from the outlet communication module to the respective electronic device, in response to the cryptographic exchange.
28. The method of any of clauses 21 to 27, further comprising updating an indicator associated with each power receptacle based on a status of the power receptacle.
29. The method of any of clauses 21 to 28, further comprising exchanging data between the outlet communication module and each of the first electronic device and the second electronic device over the localized wireless network.
30. The method of any of clauses 21 to 29, further comprising exchanging data between the first electronic device and the second electronic device over the localized wireless network.
31. The method of any of clauses 21 to 30, wherein at least one of the first electronic device and the second electronic device includes a medical device.
32. The method of any of clauses 21 to 31, wherein each power receptacle includes a power receptacle in a healthcare facility.
33. The method of any of clauses 21 to 32, wherein each power receptacle includes an alternating current power receptacle.
34. A localized wireless network, the network comprising:
   a first medical device,
   a first power receptacle configured to provide power via a cord to the first medical device, the first power receptacle being associated with a wireless communicator to provide wireless communication between the first power receptacle and the first medical device,
   a second medical device, and
   a second power receptacle configured to provide power via a cord to the second medical device, the second power receptacle being associated with a wireless communicator to provide wireless communication between the second power receptacle and the second medical device,
   wherein the network is configured such that the first medical device communicates wirelessly with the second medical device.
35. A localized wireless network, the network comprising:
   a first medical device having at least one sensor for acquiring data related to a patient,
   a first power receptacle configured to provide power via a cord to the first medical device, the first power receptacle being associated with a wireless communicator to provide wireless communication between the first power receptacle and the first medical device,

   a second medical device, and
   a second power receptacle configured to provide power via a cord to the second medical device, the second power receptacle being associated with a wireless communicator to provide wireless communication between the second power receptacle and the second medical device,
   wherein the network is configured such that the first medical device communicates wirelessly with the second medical device, wherein the first medical device is configured to publish the data related to the patient on the network, and wherein the second medical device is configured to receive the data from the network.
36. The localized wireless network of clause 35, wherein the second medical device is connected to an electronic medical record.
37. The localized wireless network of clause 36, wherein the second medical device is wirelessly connected to the electronic medical record.
38. The localized wireless network of clause 36 or 37, wherein the second medical device includes a scanner configured to scan a patient code to connect to the electronic medical record.
39. The localized wireless network of clause 38, wherein the electronic medical record is specifically linked to the patient via the patient code.
40. The localized wireless network of clause 38 or 39, wherein the patient code is provided on a patient wristband worn by the patient.
41. The localized wireless network of any of clauses 36 to 40, wherein the second medical device is configured to publish the data to the electronic medical record.
42. The localized wireless network of any of clauses 35 to 41, wherein the first medical device includes a patient support apparatus.
43. The localized wireless network of any of clauses 35 to 42, wherein the second medical device includes at least one of a physiological monitor and an infusion pump.
44. The localized wireless network of any of clauses 35 to 43, wherein the data includes services provided by the first medical device.
45. The localized wireless network of clause 44, wherein the services include at least one of an electronic medical record channel, a service-oriented device connectivity communication channel, nurse call capabilities, and priority nurse call capabilities.
46. The localized wireless network of any of clauses 35 to 45, wherein the data includes at least one of patient weight, patient identification, patient blood pressure, patient heart rate, patient respiratory rate, lighting status, caregiver presence indication, and caregiver identification.
47. The localized wireless network of any of clauses 35 to 46, wherein each of the first medical device and the second medical device operates an application programming interface to communicate over the network.
48. The localized wireless network of any of clauses 35 to 47, wherein:
   the first power receptacle includes a first power receptacle communication module that communicates with the first medical device, and
   the second power receptacle includes a second power receptacle communication module that communicates with the second medical device.
49. The localized wireless network of clause 48, wherein the first power receptacle communication module communicates with the second power receptacle communication module.
50. The localized wireless network of clause 48 or 49, wherein the first power receptacle communication module wirelessly communicates with the second power receptacle communication module.
51. The localized wireless network of any of clauses 48 to 50, wherein:
   the first power receptacle communication module wirelessly communicates with the first medical device, and
   the second power receptacle communication module wirelessly communicates with the second medical device.
52. A method of providing communication between a first medical device and a second medical device over a localized wireless network, the method comprising:
   providing power via a cord from a first power receptacle to the first medical device, the first power receptacle being associated with a wireless communicator to provide wireless communication between the first power receptacle and the first medical device, the first medical device having at least one sensor for acquiring data related to a patient,
   providing power via a cord from a second power receptacle to the second medical device, the second power receptacle being associated with a wireless communicator to provide wireless communication between the second power receptacle and the second medical device, wherein the network is configured such that the first medical device communicates wirelessly with the second medical device,
   publishing the data from the first medical on the network, and
   receiving the data at the second medical device from the network.
53. The method of clause 52, further comprising connecting the second medical device to an electronic medical record.
54. The method of clause 52 or 53, further comprising wirelessly connecting the second medical device to the electronic medical record.
55. The method of clause 53 or 54, further comprising scanning a patient code to connect the second medical device to the electronic medical record.
56. The method of clause 55, wherein the electronic medical record is specifically linked to the patient via the patient code.
57. The method of clause 55 or 56, wherein the patient code is provided on a patient wristband worn by the patient.
58. The method of any of clauses 53 to 57, further comprising publishing the data from the second medical device to the electronic medical record.
59. The method of any of clauses 52 to 58, wherein the first medical device includes a patient support apparatus.
60. The method of any of clauses 52 to 59, wherein the second medical device includes at least one of a physiological monitor and an infusion pump.
61. The method of any of clauses 52 to 60, wherein the data includes services provided by the first medical device.
62. The method of clause 61, wherein the services include at least one of an electronic medical record channel, a service-oriented device connectivity communication channel, nurse call capabilities, and priority nurse call capabilities.
63. The method network of any of clauses 52 to 62, wherein the data includes at least one of patient weight, patient identification, patient blood pressure, patient heart rate, patient respiratory rate, lighting status, caregiver presence indication, and caregiver identification.
64. The method of any of clauses 52 to 63, further comprising operating an application programming interface on each of the first medical device and the second medical device to communicate over the network.
65. The method of any of clauses 52 to 64, wherein:
   the first power receptacle includes a first power receptacle communication module that communicates with the first medical device, and
   the second power receptacle includes a second power receptacle communication module that communicates with the second medical device.
66. The method of clause 65, wherein the first power receptacle communication module communicates with the second power receptacle communication module.
67. The method of clause 65 or 66, wherein the first power receptacle communication module wirelessly communicates with the second power receptacle communication module.
68. The method of any of clauses 65 to 67, wherein:
   the first power receptacle communication module wirelessly communicates with the first medical device, and
   the second power receptacle communication module wirelessly communicates with the second medical device.
69. A method of populating an EMR computer with bed status data of a patient bed located in a patient room of a healthcare facility, the method comprising
   establishing a communication channel between a medical device other than the patient bed and the EMR computer, the medical device being located in the patient room with the patient bed,
   communicating the bed status data from the patient bed to a stationary unit located in the patient room with the patient bed,
   communicating the bed status data from the stationary unit to the medical device, and
   communicating the bed status data from the medical device to the EMR computer.
70. The method of clause 69, wherein establishing the communication channel further comprises establishing a long range wireless communication link between the medical device and a wireless access point of a network of the healthcare facility.
71. The method of clause 70, wherein the long range wireless communication link includes a Wi-Fi communication link.
72. The method of any of clauses 69 to 71, wherein communicating the bed status data from the patient bed to the stationary unit further comprises communicating the bed status data using short range wireless communication technology.
73. The method of clause 72, wherein the short range wireless communication technology includes Bluetooth communication technology.
74. The method of any of clauses 69 to 73, wherein communicating the bed status data from the stationary unit to the medical device further comprises communicating the bed status data using short range wireless communication technology.
75. The method of clause 74, wherein the short range wireless communication technology includes Bluetooth communication technology.
76. The method of any of clauses 69 to 75, wherein the medical device includes a physiological monitor.
77. The method of clause 76, wherein the physiological monitor includes one or more of a heart rate monitor, a respiration monitor, a thermometer, and a blood pressure measuring device.
78. The method of any of clauses 69 to 77, wherein the medical device comprises an infusion pump.
79. The method of any of clauses 69 to 78, wherein the medical device uses at least some of the bed status data in connection with operating a function of the medical device.
80. The method of clause 79, wherein the at least some of the bed status data includes patient weight data as detected by a weigh scale system of the patient bed.
81. The method of any of clauses 69 to 80, further comprising establishing a local mesh network in the patient room between the patient bed, the medical device, and the stationary unit in response to plugging in AC power cords from the patient bed and the medical device into respective AC receptacles of the stationary unit.
82. The method of any of clauses 69 to 81, further comprising sending a subscribe message from the medical device to the patient bed to subscribe to the bed status data.
83. The method of any of clause 82, wherein the subscribe message includes a wireless subscribe message sent directly from the medical device to the patient bed.
84. The method of clause 82 or 83, wherein the subscribe message is sent to the patient bed via the stationary unit.
85. The method of any of clauses 82 to 84, wherein the patient bed publishes the bed data for receipt by the medical device only after receiving the subscribe message.
86. The method of any of clauses 69 to 85, wherein the stationary unit is communicatively coupled to a nurse call system that receives the bed status data.
87. The method of clause 86, further comprising receiving medical device status data at the stationary unit and communicating the medical device status data to the nurse call system so that the nurse call system receives both the bed status data and the medical device status data.
88. A method comprising:
   pairing a patient bed in a patient room with a wall unit in the patient room for wireless communication between the patient bed and the wall unit,
   pairing a medical device in the patient room with the wall unit for wireless communication with the wall unit,
   communicating bed status data from the patient bed to the wall unit,
   communicating the bed status data from the wall unit to the medical device, and
   operating a function of the medical device based on the bed status data.
89. The method of clause 88, wherein communicating the bed status data from the patient bed to the wall unit further comprises communicating the bed status data using short range wireless communication technology.
90. The method of clause 89, wherein the short range wireless communication technology includes Bluetooth communication technology.
91. The method of any of clauses 88 to 90, wherein communicating the bed status data from the wall unit to the medical device further comprises communicating the bed status data using short range wireless communication technology.
92. The method of clause 91, wherein the short range wireless communication technology includes Bluetooth communication technology.
93. The method of any of clauses 88 to 92, wherein the medical device includes a physiological monitor.
94. The method of clause 93, wherein the physiological monitor includes one or more of a heart rate monitor, a respiration monitor, a thermometer, and a blood pressure measuring device.
95. The method of any of clauses 88 to 94, wherein the medical device comprises an infusion pump.
96. The method of any of clauses 88 to 95, wherein at least some of the bed status data includes patient weight data as detected by a weigh scale system of the patient bed.
97. The method of any of clauses 88 to 96, wherein pairing the patient bed in the patient room with the wall unit and pairing the medical device in the patient room with the wall unit further comprises establishing a local mesh network in the patient room between the patient bed, the medical device, and the wall unit in response to plugging in AC power cords from the patient bed and the medical device into respective AC receptacles of the wall unit.
98. The method of any of clauses 88 to 97, further comprising sending a subscribe message from the medical device to the patient bed to subscribe to the bed status data.
99. The method of clause 98, wherein the subscribe message includes a wireless subscribe message sent directly from the medical device to the patient bed.
100. The method of clause 98 or 99, wherein the subscribe message is sent to the patient bed via the wall unit.
101. The method of any of clauses 98 to 100, wherein the patient bed publishes the bed status data for receipt by the medical device only after receiving the subscribe message.
102. The method of any of clauses 88 to 101, wherein the wall unit is communicatively coupled to a nurse call system that receives the bed status data.
103. The method of clause 102, further comprising receiving medical device status data at the wall unit and communicating the medical device status data to the nurse call system so that the nurse call system receives both the bed status data and the medical device status data.
104. A localized wireless network, the network comprising:
   a first medical device having at least one sensor for acquiring data related to a patient,
   a first power receptacle configured to provide power via a cord to the first medical device, the first power receptacle being associated with a wireless communicator to provide wireless communication between the first power receptacle and the first medical device,
   a second medical device, and
   a second power receptacle configured to provide power via a cord to the second medical device, the second power receptacle being associated with a wireless communicator to provide wireless communication between the second power receptacle and the second medical device,
   wherein the network is configured such that the first medical device communicates wirelessly with the second medical device, wherein the first medical device is configured to publish the data related to the patient on the network, and wherein the second medical device is configured to receive the data from the network.
105. The localized wireless network of clause 104, wherein the second medical device is connected to an electronic medical record.
106. The localized wireless network of clause 104 or 105, wherein the second medical device is wirelessly connected to the electronic medical record.
107. The localized wireless network of any of clauses 103 to 106, wherein the second medical device includes a scanner configured to scan a patient code to connect to the electronic medical record.
108. The localized wireless network of clause 107, wherein the electronic medical record is specifically linked to the patient via the patient code.
109. The localized wireless network of clause 107 or 108, wherein the patient code is provided on a patient wristband worn by the patient.
110. The localized wireless network of any of clauses 105 to 109, wherein the second medical device is configured to publish the data to the electronic medical record.
111. The localized wireless network of any of clauses 103 to 110, wherein the first medical device includes a patient support apparatus.
112. The localized wireless network of any of clauses 103 to 111, wherein the second medical device includes at least one of a physiological monitor and an infusion pump.
113. The localized wireless network of any of clauses 103 to 112, wherein the data includes services provided by the first medical device.
114. The localized wireless network of clause 113, wherein the services include at least one of an electronic medical record channel, a service-oriented device connectivity communication channel, nurse call capabilities, and priority nurse call capabilities.
115. The localized wireless network of any of clauses 103 to 114, wherein the data includes at least one of patient weight, patient identification, patient blood pressure, patient heart rate, patient respiratory rate, lighting status, caregiver presence indication, and caregiver identification.
116. The localized wireless network of any of clauses 103 to 115, wherein each of the first medical device and the second medical device operates an application programming interface to communicate over the network.
117. The localized wireless network of any of clauses 103 to 116, wherein:
   the first power receptacle includes a first power receptacle communication module that communicates with the first medical device, and
   the second power receptacle includes a second power receptacle communication module that communicates with the second medical device.
118. The localized wireless network of clause 117, wherein the first power receptacle communication module communicates with the second power receptacle communication module.

Any theory, mechanism of operation, proof, or finding stated herein is meant to further enhance understanding of principles of the present disclosure and is not intended to make the present disclosure in any way dependent upon such theory, mechanism of operation, illustrative embodiment, proof, or finding. It should be understood that while the use of the word preferable, preferably or preferred in the description above indicates that the feature so described can be more desirable, it nonetheless cannot be necessary and embodiments lacking the same can be contemplated as within the scope of the disclosure, that scope being defined by the claims that follow.

In reading the claims it is intended that when words such as "a," "an," "at least one," "at least a portion" are used there is no intention to limit the claim to only one item unless specifically stated to the contrary in the claim. When the language "at least a portion" and/or "a portion" is used, the item can include a portion and/or the entire item unless specifically stated to the contrary. It should be understood that only selected embodiments have been shown and described and that all possible alternatives, modifications, aspects, combinations, principles, variations, and equivalents that come within the spirit of the disclosure as defined herein or by any of the following claims are desired to be protected. While embodiments of the disclosure have been illustrated and described in detail in the drawings and foregoing description, the same are to be considered as illustrative and not intended to be exhaustive or to limit the disclosure to the precise forms disclosed. Additional alternatives, modifications and variations can be apparent to those skilled in the art. Also, while multiple inventive aspects and principles have been presented, they need not be utilized in combination, and many combinations of aspects and principles are possible in light of the various embodiments provided above.

## Claims

1. A localized wireless network, the network comprising:
a first medical device having at least one sensor for acquiring data related to a patient,
a first power receptacle configured to provide power via a cord to the first medical device, the first power receptacle being associated with a wireless communicator to provide wireless communication between the first power receptacle and the first medical device,
a second medical device, and
a second power receptacle configured to provide power via a cord to the second medical device, the second power receptacle being associated with a wireless communicator to provide wireless communication between the second power receptacle and the second medical device,
wherein the network is configured such that the first medical device communicates wirelessly with the second medical device, wherein the first medical device is configured to publish the data related to the patient on the network, and wherein the second medical device is configured to receive the data from the network.

2. The localized wireless network of claim 1, wherein the second medical device is connected to an electronic medical record.

3. The localized wireless network of claim 1 or 2, wherein the second medical device is wirelessly connected to the electronic medical record.

4. The localized wireless network of any preceding claim, wherein the second medical device includes a scanner configured to scan a patient code to connect to the electronic medical record.

5. The localized wireless network of claim 4, wherein the electronic medical record is specifically linked to the patient via the patient code.

6. The localized wireless network of claim 4 or 5, wherein the patient code is provided on a patient wristband worn by the patient.

7. The localized wireless network of any preceding claim, wherein the second medical device is configured to publish the data to the electronic medical record.

8. The localized wireless network of any preceding claim, wherein the first medical device includes a patient support apparatus.

9. The localized wireless network of any preceding claim, wherein the second medical device includes at least one of a physiological monitor and / or an infusion pump.

10. The localized wireless network of any preceding claim, wherein the second medical device is configured to send a subscribe message to the first medical device to subscribe to the data.

11. The localized wireless network of any preceding claim, wherein the data includes services provided by the first medical device.

12. The localized wireless network of claim 11, wherein the services include at least one of an electronic medical record channel, a service-orientated device connectivity communication channel, nurse call capabilities, and priority nurse call capabilities.

13. The localized wireless network of any preceding claim, wherein the at least one sensor includes at least one of: a load cell; a blood pressure measuring device; a heart rate monitor; and a respiratory rate monitor.

14. The localized network of any preceding claim, wherein:
the first power receptacle includes a first power receptacle communication module that communicates with the first medical device, and
the second power receptacle includes a second power receptacle communication module that communicates with the second medical device.

15. The localized wireless network of claim 14, wherein the first power receptacle communication module communicates with the second power receptacle communication module.
